# EUROPEAN PATENT SPECIFICATION

(11) **EP 3 401 289 B1**
(45) Date of publication and mention of the grant of the patent: **05.02.2020**
(21) Application number: 16883418.2
(22) Date of filing: 22.12.2016
(51) Int. Cl.: A61L 24/02, A61L 24/00, C04B 12/02, C04B 28/34, C04B 111/00

(54) **MAGNESIUM PHOSPHATE BONE CEMENT**
MAGNESIUMPHOSPHATKNOCHENZEMENT
CIMENT OSSEUX À BASE DE PHOSPHATE DE MAGNÉSIUM

(30) Priority: 06.01.2016 CN 201610003213
(43) Date of publication of application: 14.11.2018
(73) Proprietor: Ningbo Hicren Biotechnology Co., Ltd., Ningbo, Zhejiang 315336 (CN)
(72) Inventor: LV, Shiwen, Ningbo Zhejiang 315336 (CN); MAO, Keya, Ningbo Zhejiang 315336 (CN); ZHUO, Qingshan, Ningbo Zhejiang 315336 (CN); ZHANG, Pengyun, Ningbo Zhejiang 315336 (CN)
(74) Representative: J A Kemp LLP
(86) International application number: PCT/CN2016/111415
(87) International publication number: WO 2017/118292

(56) References cited:
- EP-A1- 2 545 944
- CN-A- 1 307 908
- CN-A- 101 157 045
- CN-A- 103 007 340
- CN-A- 103 007 340
- US-A1- 2009 136 589
- US-A1- 2015 314 045
- US-B1- 6 533 821

## Description

### RELATED APPLICATION

This present invention application claims the benefit of priority of Chinese Patent Application No. 201610003213.9, filed on January 6, 2016 and entitled "Magnesium Phosphate Bone Cement".

### TECHNICAL FIELD

The present invention relates to the field of medical biomaterials, more especially, to a magnesium phosphate bone cement.

### BACKGROUND

According to the report from the Ministry of Civil Affairs, there are nearly three million people with bone defects and bone injuries in China each year, and the demand for bone repairing materials is huge. Currently, the bone repairing materials used clinically in the field of orthopedics mainly include medical biomaterials such as calcium sulfate, calcium phosphate, and poly (methylmethacrylate) (PMMA).

Although calcium phosphate has good biocompatibility and biological activity, it degrades very slowly in vivo due to the high calcium-to-phosphorus ratio. For example, Chinese Patent No. 200810092798.1 discloses a calcium phosphate-based bone cement, which has a good biocompatibility, yet has too slow degradation rate and poorer adhesion.

In recent years, there have been many researches on calcium phosphate-based composite biomaterials. For example, U.S. Patent No. 6,331,312 discloses an injectable calcium phosphate-based composite material that can be used as a bone filler and an adhesion agent. Although it is degradable and absorptive, it has lower strength, and its adhesive force is still unsuitable for connecting ligaments with bone tissues. Chinese patent No. 201010534490.5 discloses an injectable degradable calcium sulfate and calcium phosphate composite material, which has good osteoconductivity and biocompatibility, yet has defects such as poorer adhesive force, etc..

PMMA bone cement is often applied in the fixation of artificial prosthetic joint because of its easy formability and good cohesiveness. For example, U.S. Patent No. 5,968,999 describes a bone adhesive PMMA used for orthopedics, but PMMA releases a large amount of heat during the process of solidification, which may result in necrosis of tissues; what's more, PMMA shrinks during solidification. Moreover, PMMA is not degradable and absorbable, and has poorer biocompatibility.

In the prior art, some scholars have conducted some researches on magnesium-based bone cement. For example, Chinese Patent No. 201010205094.8 discloses a product for forming injectable calcium magnesium bone cement, which comprises a solid phase powder obtained by evenly mixing composite calcium phosphate with magnesium phosphate powder, and curing liquid obtained by dissolving dextrin and hydrophosphate in water. Chinese Patent No. 01105373.9 discloses an inorganic bone adhesive agent, which comprises an alkaline compound, phosphate, calcium phosphate bone cement, and retarder, and which is used for fixing the artificial joints, fixing screws, and bonding and fixing broken bone pieces.

US 6,533,821 discusses a bio-adhesive derived from a mixture comprising KH₂PO₄, a metal oxide, a calcium containing compound and water.

US 2009/136589 discusses an adhesive composition for sealing lung tissue comprising KH₂PO₄, metal oxide, a calcium compound and water, wherein the weight percentage ratio of KH₂PO₄ and the oxide is at least approximately 1:0.5.

US 2015/314045 discusses a bio-material composition which may be used for fusing objects to bone, said material comprising magnesia, potassium biphosphate, a calcium phosphate and water.

CN 103007340 discusses a bone repair material comprising a solid-phase powder and a liquid phase, wherein the solid-phase powder contains 5-20% of tricalcium silicate, 30-70% of electric smelting magnesium oxide, 5-30% of calcium phosphate bone cement, 5-30% of potassium dihydrogen phosphate or ammonium dihydrogen phosphate, and retarder accounting for 2-10% of the weight of basic oxide.

Some problems still exist in the actual application process of the existing bone cement: 1. When developed as bone adhesion agent, the material still lags behind the normal healing rate of fractures while ensuring a stable connection, which hinders the healing of the fractures. 2. How to further improve the biocompatibility of materials. As seen from the existing research results, these materials still cause inflammatory reactions to some extent, which have negative effects on the healing of tissues. For example, the degradation of potassium salt-typed MPC in body will cause the abnormal increase of ions (such as potassium ions and magnesium ions) in local body fluids and lead to alkalinization caused by the increase of pH value. This kind of degradation result has the tendency of causing local acid-base imbalance and local electrolyte disturbances in the body, resulting in adverse effects on the healing of tissues.

### SUMMARY

In view of this, an objective of the present invention is to provide a magnesium phosphate bone cement with a better biocompatibility. To achieve this objective, a silicon-containing compound is added to the magnesium phosphate bone cement of the present invention. In the process of degradation, a porous gel layer rich in Si-O-Si is generated on the surface of the bone cement. A large number of negatively charged Si-OH are generated on the surface of the silicone gel layer, and bond together with various kinds of protein through hydrogen bonds and ionic amine bonds (-Si-O-H₃N⁺-) to form a high density protein adsorption. The silica gel layer and the hydroxyapatite layer formed on the surface thereof have a large surface area suitable for adsorbing a large number of biomolecules, thereby promoting extracellular response. In addition, the silicone gel layer can chelate with metal ions such as magnesium ions, thereby reducing the ionization degree of metal ions in body fluids, reducing the toxicity caused by these abnormally increased ions, and improving biocompatibility.

Another objective of the present invention is to provide a bone cement whose degradation rate matches with the new bone growth. By adjusting the proportion of ammonium dihydrogen phosphate in the phosphate and controlling the amount of ammonia produced by the reaction, magnesium phosphate bone cements with different porosity and pore sizes are obtained. These pores can increase the contact area of the magnesium phosphate bone cement with the body fluid and accelerate the rate of degradation. Additionally, the pores help cells crawling, and can promote cells adhering, cells breeding and cells differentiation with a certain osteoinduction.

The technical scheme adopted is as follows:
A magnesium phosphate bone cement comprises powder agent and liquid agent; a liquid-to-solid ratio of the liquid agent and the powder agent is 0.1-0.5 ml/g, and the powder agent comprises following components: Phosphate accounting for 32-70 wt%; magnesium oxide accounting for 28-65 wt%; Silicon-containing compound accounting for 1-15 wt%.

The objective of the present invention can be further achieved by the technical schemes as follows:
The Phosphate consists of (a) one of or a mixture of two or more of potassium dihydrogen phosphate, sodium dihydrogen phosphate, and calcium dihydrogen phosphate and (b)
ammonium dihydrogen phosphate, and the ammonium dihydrogen phosphate accounts for 5-30 wt% of a total weight of the phosphate.

Preferably, the magnesium oxide is obtained by calcining magnesium hydroxide and/or basic magnesium carbonate; the calcination temperature in the calcination process is 1200 °C-1600 °C, and the calcination time is 1-6 hours.

Different calcination processes affect the activity of magnesium oxide, thereby affecting the strength and the solidification time of the bone cement.

More preferably, the calcination temperature in said calcination process is 1400 °C -1500 °C, and the calcination time is 3-4 hours.

Additionally, appropriate amount of degradable and adhesion-promoting materials are added into the magnesium phosphate bone cement in the present invention, to further improve the cohesiveness of the bone cement.

Preferably, the degradable and adhesion-promoting materials comprise sugar or derivatives thereof.

Different sugars have different dissolution rates in water, and have different effects on the improvement of the cohesiveness of the bone cement.

Preferably, the sugar is disaccharide, and the disaccharide is sucrose.

Preferably, the degradable and adhesion-promoting material comprises at least one of beeswax, sodium hyaluronate, and bone glue.

Preferably, the silicon-containing compound comprises silica and/or silicate.

Preferably, the silicate comprises silicate glass, and the silicate glass comprises one of or a mixture of two or more of 45S5, 45S5.4F, 52S4.6, 55S4.3, 60S3.8, 42SF, 46SF, 49SF, 52SF, 55SF, and 60SF.

For easier practical applications and clinical operations, an appropriate amount of retarder is added in the bone cement.

Preferably, the retarder comprises one of or a mixture of two or more of boric acid, borate, citric acid, and citrate.

Preferably, the liquid agent comprises one of or a mixture of two or more of deionized water, sodium chloride solution, and phosphoric acid solution.

Preferably, diameters of over 90% of the particles in the powder agent range from 1 to 75 µ m, and diameters of less than 3% of the particles are greater than 100 µm.

Compared with the prior art, the present invention has following beneficial effects:
1. The magnesium phosphate bone cement of the present invention can be degraded and absorbed. In the process of degradation, a porous gel layer rich in Si-O-Si is generated on the surface of the bone cement, and a high density protein adsorption is formed, thereby promoting extracellular response. The silica gel layer can chelate with metal ions such as magnesium ions, thereby reducing the ionization degree of the metal ions in the body fluid, reducing the toxicity caused by these abnormally increased ions, and improving biocompatibility of the magnesium phosphate bone cement. The magnesium phosphate bone cement can be widely used in clinical bone repairs and so on..
2. The degradable and adhesion-promoting materials in the magnesium phosphate bone cement of the present invention, such as beeswax, sodium hyaluronate and so on, can enhance the adhesive force of the bone cement of the present invention. What's more, the magnesium phosphate bone cement releases heat at a low temperature in the process of solidification; its volume is slightly expanded after having solidified, and it is inlaid into the bone tissues, making the combination of the magnesium phosphate bone cement and the bone tissues stronger. The magnesium phosphate bone cement can be used for bonding bones or strengthening the fixation of the steel plates and screws, etc..
3. The magnesium phosphate bone cement of the present invention has a certain porosity after having solidified, and the porosity is adjustable. The pores help the cell crawling, and can promote cells adhering, cells breeding and cells differentiation, with a certain degree of osteoinduction. Additionally, the pores accelerate the degradation rate of the magnesium phosphate bone cement and make the magnesium phosphate bone cement better match with the growth of the bone tissues.
4. The magnesium phosphate bone cement of the present invention has performance of a high strength in early stage. In the process of degradation, the bone cement can maintain a strength higher than that of the cancellous bone. The bone cement can be applied in transporting growth factors and other drugs and controlling the release rate thereof.

### BRIEF DESCRIPTION OF THE DRAWINGS

In order to make the content of the present disclosure more clear and better understood, the present disclosure will be further described in more details with reference to the accompanying figures and embodiments, wherein,
Fig. 1 shows a large amount of bone-like hydroxyapatite deposition on the surface of the bone cement of the present disclosure when being soaked in the simulated body fluid (SBF);
Fig. 2 shows the change of compressive strength of the bone cement of the present disclosure after being soaked in the simulated body fluid (SBF) for different periods of time;
Fig. 3 shows a silicone gel layer produced on the surface of the bone cement of the present disclosure after being soaked in the simulated body fluid (SBF);
Fig. 4 is a Micro-CT image of the bone cement of the present disclosure one month after the bone cement being used in a fracture bonding surgery;
Fig. 5 is a Micro-CT image of the bone cement of the present disclosure three months after the bone cement being used in a fracture bonding surgery;
Fig. 6 is a Micro-CT image of the bone cement of the present disclosure six months after the bone cement being used in fracture bonding surgery.

### DETAILED DESCRIPTION OF THE PREFERRED EMBODIMENTS

To make the objectives, technical solutions, and advantages of the present invention clearer, the present disclosure will be further described in more details with reference to the accompanying figures and embodiments.

The magnesium oxide of the present invention is obtained by calcining magnesium hydroxide and/or basic magnesium carbonate. The calcination temperature in the calcination process is 1200 °C-1600 °C, and the calcination time is 1-6 hours. Preferably, the calcination temperature is 1400 °C -1500 °C, and the calcination time is 3-4 hours. Different calcination processes affect the activity of magnesium oxide, thereby affecting the strength and the solidification time of the bone cement.

Exemplary formulations of the bone cement disclosed herein are as follows:

### Reference Formulation a:

Formulate powder agent according to the following weight percentage (wt%):

| | |
|---|---|
| KH₂PO₄ | 45%; |
| MgO | 41%; |
| SiO₂ | 8%; |
| Sucrose | 4%; |
| β-Tcp | 1%; |
| Na₂B₄O₇ | 1%; |

The liquid agent is deionized water, and the liquid-to-solid ratio of the powder agent and the liquid agent is 0.2 ml/g.

### Formula b:

Formulate powder agent according to the following weight percentage (wt%):

| | |
|---|---|
| Ca(H₂PO₄)₂ | 40%; |
| MgO | 45%; |
| NH₄H₂PO₄ | 5%; |
| 45S5 | 5%; |
| Glucose | 4.5%; |
| H₃BO₃ | 0.5%; |

The liquid agent is physiological saline, and the liquid-solid ratio of the powder agent and the liquid agent is 0.22 ml/g.

### Formula c:

Formulate powder agent in the following weight percentage (wt%):

| | |
|---|---|
| NaH₂PO₄ | 40%; |
| MgO | 40%; |
| NH₄H₂PO₄ | 5%; |
| Na₂SiO₃ | 8%; |
| Bone glue | 6%; |
| Na₃C₆H₅O₇ | 1%; |

The liquid agent is phosphoric acid solution, and the liquid-to-solid ratio of the powder agent and the liquid agent is 0.25 ml/g.

### Formula d:

Formulate powder agent according to the following weight percentage (wt%):

| | |
|---|---|
| KH₂PO₄ | 35%; |
| MgO | 50%; |
| NH₄H₂PO₄ | 5%; |
| 60S3.8 | 7%; |
| Sucrose | 2%; |
| Na₂B₄O₇ | 1%; |

The liquid agent is deionized water, and the liquid-solid ratio of the powder agent and the liquid agent is 0.16 ml/g.

### Formula e:

Formulate powder agent according to the following weight percentage (wt%):

| | |
|---|---|
| NaH₂PO₄ | 30%; |
| MgO | 51%; |
| NH₄H₂PO₄ | 8%; |
| 46SF | 10%; |
| Sodium hyaluronate | 0.5%; |
| Na₂B₄O₇ | 0.5%; |

The liquid agent is physiological saline, and the liquid-solid ratio of the powder agent and the liquid agent is 0.32 ml/g.

### Formula f:

Formulate powder agent according to the following weight percentage (wt%):

| | |
|---|---|
| KH₂PO₄ | 35%; |
| MgO | 39%; |
| NH₄H₂PO₄ | 8%; |
| SiO₂ | 8%; |
| Sucrose | 4%; |
| Na₂B₄O₇ | 1%; |

The liquid agent is deionized water, and the liquid-solid ratio of the powder agent and the liquid agent is 0.19 ml/g.

### Formula g:

Formulate powder agent according to the following weight percentage (wt%):

| | |
|---|---|
| Ca(H₂PO₄)₂ | 35%; |
| MgO | 45%; |
| NH₄H₂PO₄ | 10%; |
| 45S5 | 8%; |
| Beeswax | 1.5%; |
| H₃BO₃ | 0.5%; |

The liquid agent is physiological saline, and the liquid-solid ratio of the powder agent and the liquid agent is 0.23 ml/g.

### Formula h:

Formulate powder agent according to the following weight percentage (wt%):

| | |
|---|---|
| KH₂PO₄ | 36%; |
| MgO | 44%; |
| NH₄H₂PO₄ | 5%; |
| Na₂SiO₃ | 8%; |
| Maltose | 6.5%; |
| Na₃C₆H₅O₇ | 0.5%; |

The liquid agent is phosphoric acid solution, and the liquid-to-solid ratio of the powder agent and the liquid agent is 0.28 ml/g.

### Formulation i:

Formulate powder agent according to the following weight percentage (wt%):

| | |
|---|---|
| KH₂PO₄ | 45%; |
| MgO | 40%; |
| NH₄H₂PO₄ | 5%; |
| 45S5 | 5%; |
| Sucrose | 4%; |
| Na₂B₄O₇ | 1%; |

The liquid agent is deionized water, and the liquid-solid ratio of the powder agent and the liquid agent is 0.16 ml/g.

### Formula j:

Formulate powder agent according to the following weight percentage (wt%):

| | |
|---|---|
| NaH₂PO₄ | 30%; |
| MgO | 51%; |
| NH₄H₂PO₄ | 8%; |
| 55SF | 7%; |
| Cellulose | 3.5%; |
| Na₂B₄O₇ | 0.5%; |

The liquid agent is physiological saline, and the liquid-solid ratio of the powder agent and the liquid agent is 0.3 ml/g.

The above exemplary formulations are some preferred formulation ratios of the present invention and are not intended to limit the present invention.

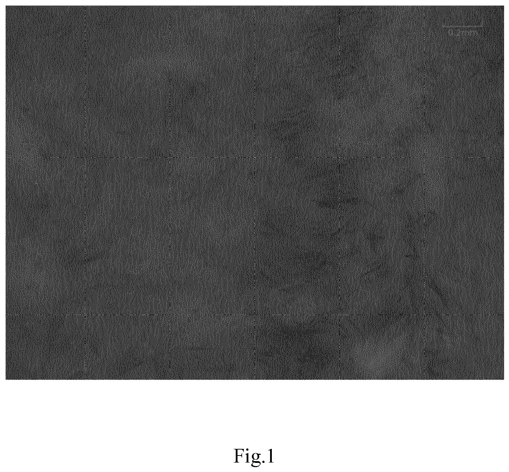

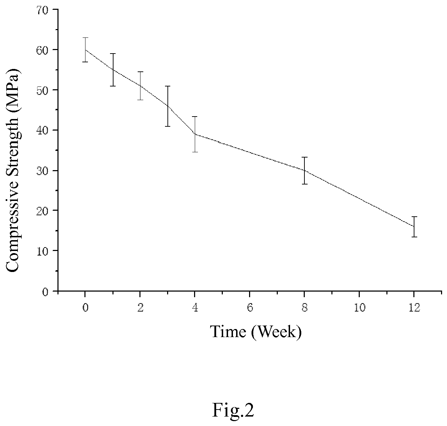

In one of the embodiments of the present invention, the powder agent and the liquid agent are blended according to formula a, and poured into a mold after being stirred for 3 minutes, and unloaded from the mold half an hour later, and then placed for 24 hours in an environment with temperature of 37° C and humidity of 100%, where the average value of the measured compressive strength reaches 60MPa. The solidified bone cement particles are soaked in the simulated body fluid (SBF). One day later, as shown in Fig. 1, a large amount of bone-like hydroxyapatite deposition is found on the surface of the bone cement particles, which indicates that the bone cement has a good biological activity. As shown in Fig. 2, after the bone cement particles are soaked for 12 weeks, the average value of the measured compressive strength is 16 MPa, which remains above the human cancellous bone strength.

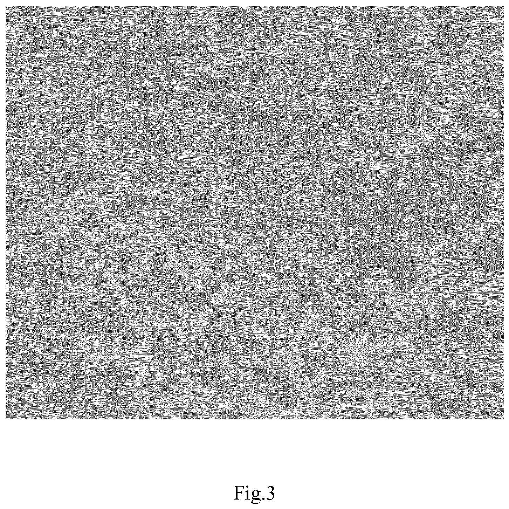

In one of the embodiments of the present invention, the powder agent and the liquid agent are blended according to the formula d, and poured into a mold after being stirred for 2 minutes, and unloaded from the mold half an hour later, and then placed for 24 hours in an environment with temperature of 37° C and humidity of 100%. The solidified bone cement is soaked in simulated body fluid (SBF), and as shown in Fig. 3, a porous silicone gel layer is formed on the surface one week later.

As a degradable bonding agent, the bone cement fixes and repairs comminuted fractures.

In one of the embodiments of the present invention, the powder agent and the liquid agent are blended according to formula h, and stirred for 3 minutes and then set aside. The experimental model is a pig shin bone fracture, and 60 fresh shin bones of pigs are serially numbered and divided into six groups A, B, C, D, E and F through CHISS software, and each group contains 10 bones. A square fracture section is produced inside each shin bone about 3 cm below the platform of the pig shin bone by a bone chisel, and the surface area of the square fracture section is trimmed to be 1cm², to facilitate the calculation of the adhesive strength after measurement. The well- blended bone cement is uniformly daubed on the cross-section of the fracture. After being fixed under pressure for 15 minutes, the groups A, B and C, and the groups D, E and F cure under the room temperature of 23° C and 37° C respectively and in the simulated body fluid. The compressive strength is measured after half an hour, two hours and 24 hours respectively. The results are as follows:

**Table 1 Comparison of average values of compressive strength after the bone cement has solidified under different conditions (x̅±s, N/cm²)**

| Solidification time | Solidification conditions | | t value | P value |
|---|---|---|---|---|
| | Room temperature of 23°C | 37°C, in simulated body fluid | | |
| 0.5h | 98.31±14.96 | 91.33±9.10 | 1.26 | 0.224 |
| 2h | 117.33±22.93 | 114.28±12.19 | 0.37 | 0.715 |
| 24h | 118.78±20.01 | 119.59±20.96 | 0.09 | 0.931 |

As can be seen from the above comparison, there is no significant difference between the average values of compressive strength of the bone cement after solidification at room temperature and in the simulated body fluid for half an hour, two hours and 24 hours respectively, and better bonding strength can be achieved with solidification at room temperature at 23°C for 2h which is almost the same time of completing a clinical surgery. Therefore, the bone cement can be applied in clinical practice to directly bond and fix fractures.

In one of the embodiments of the present invention, 15 adult healthy pigs are selected for the experimental group to carry out the bone bonding tests in vivo, and a screw sized 4.0 mm × 4.0 cm for cancellous bone is used for internal fixation in control group.

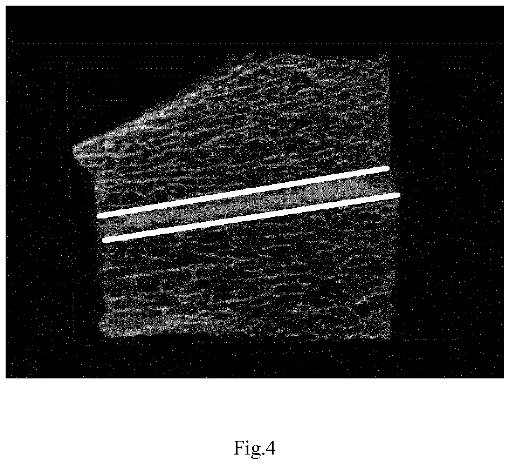

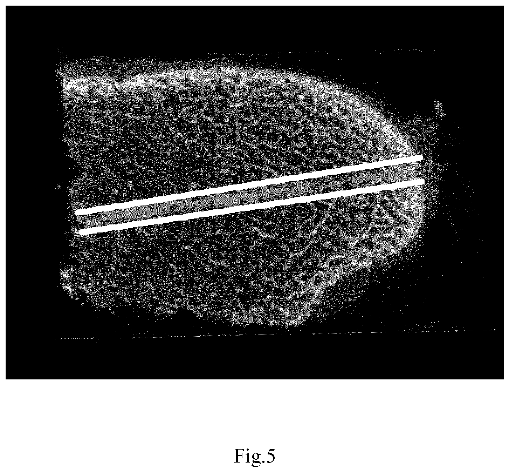

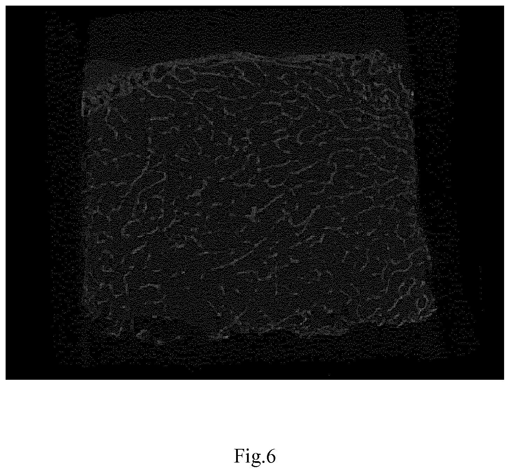

One month, 3 months, and 6 months after the surgery, two knee joints of the animals are photographed with frontal and lateral X-rays and CT, and scanned by Micro-computed tomography (Micro-CT). As shown in Figs. 4 to 6, there is no displacement of fracture in both pig experimental group and the control group. One month later, the appositions and the alignments of the fractures in the experimental group and the control group are good; the fracture line is clearly visible; and the fracture callus is normal and is calcified to form bridge fracture callus; and part of the bone trabecula penetrates the bone cement. Through comparing the images taken three months after the surgery with the images taken one month after the surgery, no displacements of the fractures are found; the fracture line is blurred; the bone cement is gradually absorbed and degraded; and the fracture has healed. As shown in the images taken six months after the surgery, the fracture line completely disappears; MPC is completely absorbed and degraded; and the fractures have completely healed.

The analyses for concentrations of serum phosphorus, serum magnesium, and serum calcium in pigs before the surgery and at three time points after the surgery are performed through the variance analysis of statistical method. The results are as follows:

**Table 2 Variance Analysis for Concentrations of Serum Phosphorus, Serum Magnesium and Serum Calcium at Different Time Points (x̅±s, mmol/L)**

| item | Group | | | | F value | P value |
|---|---|---|---|---|---|---|
| | before surgery | 1 month after surgery | 3 months after surgery | 6 months after surgery | | |
| Serum Phosphorus concentration | 2.77±0.16 | 2.64±0.15 | 2.63±0.25 | 2.55±0.39 | 0.85 | 0.483 |
| Serum Magnesium concentration | 0.94±0.11 | 0.90±0.13 | 0.92±0.10 | 0.94±0.10 | 0.26 | 0.853 |
| Serum Calcium concentration | 2.44±0.12 | 2.45±0.14 | 2.46±0.09 | 2.41±0.07 | 0.25 | 0.862 |

The serum magnesium concentration has no significant changes before the surgery, one month after the surgery, 3 months after the surgery and 6 months after the surgery, which indicates that, during fracture healing, the bone cement implanted in the pigs doesn't have adverse effects on the serum electrolyte ions concentrations; the dissolution and absorption of the bone cement can be fully balanced during the normal metabolic process of the pigs. During the experiment, no abnormal reactions such as inflammation emerged in the domestic pigs, and no pigs died due to the implantation of bone cement. Therefore, it is believed that the bone cement of the present invention can be degraded and absorbed, and that the bone cement has a high biological safety and a good biocompatibility.

The bone cement is used to strengthen the fixation of screws.

In one of the embodiments of the present invention, the powder agent and the liquid agent are blended according to formulation i, and stirred for 3 minutes and then set aside. The bone cement of the present invention is used in the experimental group, and PMMA bone cement is used in the control group. The test method is as follows: divide 18 landraces pigs weighing 50±2kg into two groups randomly; a screw track is provided at the humerus medialis epicondylus and parallel with the long axis of trochlea in the humerus; the diameter of the screw track is 8mm; and insert a screw for cancellous bone, which is 6 mm in diameter and 40 mm in length; the rest is filled with and boned by the stirred bone cement. X-ray and CT scans were taken one month, 3 months, and 6 months after the surgery, to observe the absorption of the bone cement and the growth of new bone. The animals were killed at the time points of one month, 3 months and 6 months after the surgery, so as to take samples. Some specimens were used for analyzing the diameter of the screw track, and the other specimens were used for measuring biomechanical.

Radiography of X-ray and CT scans shows that the thickness of bone cement around the screw in the experimental group gradually becomes thinner at the time points of one month, 3 months and 6 months after the surgery, and the bone cement is tightly surrounded by new bone; i.e., as time goes on, the bone cement is gradually absorbed and is replaced by new bone; the thickness of the poly(methyl methacrylate) bone cement around the screw in the control group does not change significantly; i.e., the poly(methyl methacrylate) could not be decomposed and absorbed by the body. Histological analysis shows that the diameters of the screw track in the experimental group are 8.20±0.10mm, 7.10±0.20mm, and 6.13±0.06mm for one month, 3 months, and 6 months respectively after the surgery. The diameters of the screw track at three time points are significantly different (P<0.05); in the control group, the diameters of the screw track are 8.23±0.05mm, 8.10±0.04mm, and 8.13±0.06 mm respectively, having no significant statistical difference (P>0.05). One month after the surgery, there is no significant difference between the diameters of the screw track in the experimental group and that in the control group (P>0.05). At two time points, 3 months and 6 months after the surgery, the diameter of the screw track in the experimental group is smaller than that in the control group, and there is a significant statistical difference (P<0.05).

Withdrawal forces are measured one month, three months and six months after the surgery. The maximum withdrawal forces in the experimental group are 4.96±0.43kN, 3.97±0.26kN, and 2.53±0.15kN respectively, and the maximum withdrawal forces measured at three points have a statistical difference (P<0.05); the maximum withdrawal forces in the control group are 2.80±0.36kN, 2.62±0.24kN and 3.06±0.38kN respectively, and the maximum withdrawal forces measured at three points have no significant statistical difference (P>0.05). The maximum withdrawal forces measured in the experimental group at two points, i.e., one month and 3 months after the surgery are greater than those measured one month and 3 months after the surgery in the control group, and there is a significant statistical difference (P<0.05); there is not a significant statistical difference between the maximum withdrawal force measured 6 months after the surgery in the experimental group and the maximum withdrawal force measured 6 months after the surgery in the control group (P>0.05).

The experimental results show that the bone cement of the present invention has a good biological safety and can effectively enhance the internal fixation strength. Within 3 months after the bone cement is implanted, the bone cement has a better reinforced fixation for the screw than PMMA does, and the bone cement can be mostly absorbed and decomposed within 6 months, and be replaced by autologous bone.

It is apparent that the above mentioned embodiments are only examples for clearly illustrating the present invention, but not intend to limit the embodiments of the present invention.

## Claims

1. A magnesium phosphate bone cement, comprising a powder agent and a liquid agent, wherein, a liquid-to-solid ratio of the liquid agent and the powder agent is 0.1-0.5 ml/g, and the powder agent comprises following components:
| | |
|---|---|
| phosphate | 32-70 wt%; |
| magnesium oxide | 28-65 wt%; and |
| silicon-containing compound | 1-15 wt%; |
wherein, the phosphate consists of (a) one of or a mixture of two or more of potassium dihydrogen phosphate, sodium dihydrogen phosphate, and calcium dihydrogen phosphate; and (b) ammonium dihydrogen phosphate, wherein the ammonium dihydrogen phosphate accounts for 5-30 wt% of a total weight of the phosphate;
and wherein the silicon-containing compound comprises silica and/or silicate.

2. The magnesium phosphate bone cement according to claim 1, **characterized in that**, the powder agent further comprises a degradable and adhesion-promoting material.

3. The magnesium phosphate bone cement according to claim 2, **characterized in that**, the degradable and adhesion-promoting material comprises at least one of sugar, beeswax, sodium hyaluronate, and bone glue.

4. The magnesium phosphate bone cement according to any one of claims 1 to 3, **characterized in that**, the silicate comprises silicate glass.

5. The magnesium phosphate bone cement according to any one of claims 1 to 4, **characterized in that**, the magnesium phosphate bone cement comprises a retarder, and the retarder comprises one of or a mixture of two or more of boric acid, borate, citric acid, and citrate.

6. The magnesium phosphate bone cement according to any one of claims 1 to 5, **characterized in that**, the liquid agent comprises one of or a mixture of two or more of deionized water, sodium chloride solution, and phosphoric acid solution.

## Patentansprüche

1. Magnesiumphosphatknochenzement, umfassend ein pulverförmiges Mittel und ein flüssiges Mittel, wobei ein Flüssigkeit-zu-Feststoff-Verhältnis des flüssigen Mittels und des pulverförmigen Mittels 0,1-0,5 ml/g ist und das pulverförmige Mittel die folgenden Komponenten umfasst:
| | |
|---|---|
| Phosphat | 32-70 Gew.-%; |
| Magnesiumoxid | 28-65 Gew.-%; und |
| siliziumhaltige Verbindung | 1-15 Gew.-%; |
wobei das Phosphat aus (a) einem aus oder einem Gemisch aus zwei oder mehreren aus Kaliumdihydrogenphosphat, Natriumdihydrogenphosphat und Calciumdihydrogenphosphat; und (b) Ammoniumdihydrogenphosphat besteht, wobei das Ammoniumdihydrogenphosphat 5-30 Gew.-% eines Gesamtgewichts des Phosphats ausmacht;
und wobei die siliziumhaltige Verbindung Siliziumdioxid und/oder Silikat umfasst.

2. Magnesiumphosphatknochenzement nach Anspruch 1, **dadurch gekennzeichnet, dass** das pulverförmige Mittel ferner ein abbaubares und haftungsförderndes Material umfasst.

3. Magnesiumphosphatknochenzement nach Anspruch 2, **dadurch gekennzeichnet, dass** das abbaubare und haftungsfördernde Material mindestens eines aus Zucker, Bienenwachs, Natriumhyaluronat und Knochenleim umfasst.

4. Magnesiumphosphatknochenzement nach einem der Ansprüche 1 bis 3, **dadurch gekennzeichnet, dass** das Silikat Silikatglas umfasst.

5. Magnesiumphosphatknochenzement nach einem der Ansprüche 1 bis 4, **dadurch gekennzeichnet, dass** der Magnesiumphosphatknochenzement einen Reaktionshemmer umfasst und der Reaktionshemmer eines aus oder ein Gemisch aus zwei oder mehreren aus Borsäure, Borat, Zitronensäure und Citrat umfasst.

6. Magnesiumphosphatknochenzement nach einem der Ansprüche 1 bis 5, **dadurch gekennzeichnet, dass** das flüssige Mittel eines aus oder ein Gemisch aus zwei oder mehreren aus deionisiertem Wasser, Natriumchloridlösung und Phosphorsäurelösung umfasst.

## Revendications

1. Ciment osseux à base de phosphate de magnésium, comprenant un agent poudreux et un agent liquide, dans lequel, un rapport liquide-solide de l'agent liquide et l'agent poudreux est 0,1 à 0,5 ml/g, et l'agent poudreux comprend les composants suivants :
phosphate 32 à 70 % en poids ;
oxyde de magnésium 28 à 65 % en poids ; et
composé contenant du silicium 1 à 15 % en poids ;
dans lequel le phosphate est constitué de (a) un de, ou un mélange de deux, ou plus, de : dihydrogénophosphate de potassium, dihydrogénophosphate de sodium, et dihydrogénophosphate de calcium ; et (b) dihydrogénophosphate d'ammonium, dans lequel le dihydrogénophosphate d'ammonium constitue de 5 à 30 % en poids d'un poids total du phosphate ;
et dans lequel le composé contenant du silicium comprend de la silice et/ou du silicate.

2. Ciment osseux à base de phosphate de magnésium selon la revendication 1, **caractérisé en ce que** l'agent poudreux comprend en outre un matériau dégradable et favorisant l'adhérence.

3. Ciment osseux à base de phosphate de magnésium selon la revendication 2, **caractérisé en ce que** le matériau dégradable et favorisant l'adhérence comprend au moins un de : sucre, cire d'abeille, hyaluronate de sodium, et colle osseuse.

4. Ciment osseux à base de phosphate de magnésium selon l'une quelconque des revendications 1 à 3, **caractérisé en ce que** le silicate comprend du verre de silicate.

5. Ciment osseux à base de phosphate de magnésium selon l'une quelconque des revendications 1 à 4, **caractérisé en ce que** le ciment osseux à base de phosphate de magnésium comprend un retardateur, et le retardateur comprend un de, ou un mélange de deux, ou plus, de : acide borique, borate, acide citrique, et citrate.

6. Ciment osseux à base de phosphate de magnésium selon l'une quelconque des revendications 1 à 5, **caractérisé en ce que** l'agent liquide comprend un de, ou un mélange de deux, ou plus, de : eau déionisée, solution de chlorure de sodium, et solution d'acide phosphorique.
